# EUROPEAN PATENT APPLICATION

(11) **EP 3 450 427 A1**
(43) Date of publication of application: **06.03.2019**
(21) Application number: 17788790.8
(22) Date of filing: 27.04.2017
(51) Int. Cl.: C07D 211/56, A61K 31/451, A61K 9/19, A61P 31/16

(54) **CYCLOHEXENE DERIVATIVE OR PHARMACEUTICALLY ACCEPTABLE SALT THEREOF, AND APPLICATION OF SAME**

(30) Priority: 28.04.2016 CN 201610281019
(71) Applicant: Guangzhou Henovcom Bioscience Co. Ltd., Guangzhou, Guangdong 510530 (CN)
(72) Inventor: ZHANG, Jiancun, Guangzhou Guangdong 510530 (CN); LI, Deyao, Guangzhou Guangdong 510530 (CN); LI, Hong, Guangzhou Guangdong 510530 (CN); WANG, Kun, Guangzhou Guangdong 510530 (CN); LIU, Yan, Guangzhou Guangdong 510530 (CN); WU, Yan, Guangzhou Guangdong 510530 (CN); TANG, Xing, Guangzhou Guangdong 510530 (CN); SONG, Jianbo, Guangzhou Guangdong 510530 (CN)
(74) Representative: HGF Limited
(86) International application number: PCT/CN2017/082155
(87) International publication number: WO 2017/186138

(57) **Abstract**

The present invention relates to a cyclohexene derivative or pharmaceutically acceptable salt thereof and application thereof, which belongs to the technical field of pharmaceutical technology. The cyclohexene derivative of formula I or pharmaceutically acceptable salt thereof is a new broad-spectrum anti-influenza compound, having a great inhibiting effect on influenza viruses, specifically, it has a high activity to virus strains which is drug-resistant to Oseltamivir, and may be used as a broad-spectrum anti-influenza NA inhibitor effective to Tamiflu drug-resistance, for treating the flu caused by influenza viruses, thereby it is a new broad-spectrum anti-influenza drug.

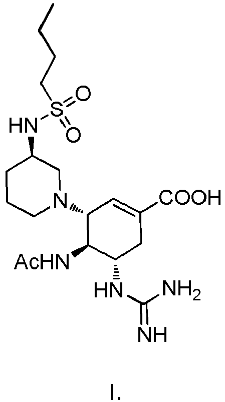

## Description

### Field of Invention

The present invention relates to the technical field of pharmaceutical technology, particularly, it relates to a cyclohexene derivative or a pharmaceutically acceptable salt thereof and use thereof.

### Background of Invention

Influenza caused by influenza viruses is one of the acute viral respiratory infectious diseases to seriously harm human health. At present, in order to control and treat domestic flu, mainly, antipyresis, analgesic or cough medicine is used for symptomatic treatment, or inactivated vaccines are vaccinated for prevention. In some countries, amantadine or rimantadine is used to treat influenza A, but the amantadine and rimantadine are both ineffective to influenza B, besides, side-effects frequently occur if the amantadine is long-term used. Now, in many countries, it is not suggested using the amantadine to treat flu any longer. In addition, due to variations of influenza viral antigen, conventional vaccines are still unable to effectively prevent flu outbreak and flu pandemic, thereby it is crucial for the research of an anti-influenza drug in flu treatments.

An influenza virus neuraminidase (NA) inhibitor is one type of the new anti-influenza drug, and has a strong inhibiting effect on influenza A, B. The NA inhibitor selectively inhibits activity of NA, to prevent progeny virions from copying and releasing in host cells, thereby effectively preventing flu and relieving symptoms. The NA inhibitor works with following principle that lipophilic groups in the NA inhibitor are combined with hydrophobic parts of the NA, then the virus will lose its capability of breaking down residues of sialic acid and glucosidic bonds between glycoproteins, blocking the copied virions from passing through a mucous layer of respiratory tract; and preventing influenza virus from continuing to spread towards surrounding cells.

Tamiflu based on principle of influenza virus NA inhibitor has been the first choice drug for preventing and treating flu all over the world. However, it has developed drug-resistance as its clinical application. Tamiflu with a common name of Oseltamivir phosphate (Oseltamivir) is a new type of anti-influenza NA inhibitor developed by Gilead company and Roche company, it has a molecular formula of C₁₆H₂₈N₂O₄.H₃PO₄, and a chemical name of ethyl (3R, 4R, 5S)-4-acetamido-5-amino-3-(1-ethylpropoxy)-1-cyclohexene-1-carboxylate phosphate (Novel selective inhibitors of viral or bacterial neuraminidases, WO9626933). Tamiflu is designed based on a three-dimensional structure of NA and can selectively inhibit the activity of influenza virus NA. It acts on conservative sites relatively stable to functional groups of NA, and can treat flu well, thus it plays an important role in prevention and treatment of flu. However, with wide clinical application of Tamiflu, the viruses frequently show resistance to it (Influenza Neuraminidase Inhibitors as Antiviral Agents. ANNUAL REPORTS IN MEDICINAL CHEMISTRY, 2006(41):287-297; In Vitro Generation of Neuraminidase Inhibitor Resistance in A(H5N1) Influenza Viruses. Antimicrob. Agents Chemother., 2009(53):4433-4440).

In recent years, influenza cases of different subtypes in different species (avian and swine influenza) indicate that influenza has been varied, and they also illustrate increasing potential harm of influenza. In 2005 year, research results of Oxford University have shown that avian influenza viruses began to resistant to Tamiflu, which is mainly caused by mutation of a H274Y portion of influenza NA. Then, Roche company urged to increase dosage of Tamiflu against avian influenza. However, the activity of Tamiflu is decreased by more than 300 times, due to the resistance caused by the mutation of H274Y, thereby it is difficult to meet the clinical requirement just by increasing dosage. A recently approved Peramivir is only limitedly effective against the H274Y mutant influenza virus.

China is a high-prevalence area of influenza and due to its ecological environment and sanitary condition, besides the situation of the drug-resistance to Tamiflu has become server; thereby it is an issue to be considered and solved by the whole society as to how to deal with the future high-risk and highly lethal influenza viruses. As the drug resistance to Tamiflu will be stronger, there is a huge market demand and social significance for the development of a new broad-spectrum anti-influenza drug that is still effective against the viruses drug-resistant to Tamiflu.

### Description of the Invention

It is therefore an objective of the present invention to provide a cyclohexene derivative or a pharmaceutically acceptable salt thereof, which is a broad-spectrum anti-influenza drug effective to drug-resistant influenza viruses.

A cyclohexene derivate of formula I or a pharmaceutically acceptable salt thereof is shown as follows:

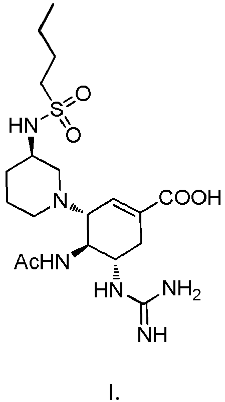

Based on previous research (Patent Applicant No. CN201310130035.2), the present inventor has made further research into some of compounds therein with better activities and screened out the compounds with better activities for observing druggabilities such as stability, process scale-up, etc. The results showed that there are some problems that some of compounds have a poor stability and are unable to meet the requirement of the druggabilities; some compounds are hypertoxic; some compounds fail to fulfill the requirement of process scale-up in the industry due to their poor physical and chemical properties, for example, (3R,4R,5S)-4-acetamido -5-guanidino-3-((S)-3- n-butylsulfonamidopiperidin)-1-cyclohexene-1-carboxylic acid has a good effect on inhibiting influenza virus and less toxic and side effects, however, such compound is semisolid at room temperature and is difficult to separate and purify, and thus it is unable to fulfill t the requirement of process scale-up in the industry.

Based on the above research, the present inventor has tried a variety of processing methods and means such as a structural modification or a excipient adding, etc, to attempt to improve stability and properties of the drug while maintaining the activity of such compound, thus to improve the druggability.

Finally, in the experiments, the inventor has accidently obtained compound of formula I, (3R,4R,5S)-4-acetamido-5-guanidino-3-((3R)-3-n-butylsulfonamidopiperidin)-1-cyclohexene-1-carboxylic acid by changing configuration of specific chiral center of the above compound of (3R,4R,5S)-4-acetamido-5-guanidino-3-((3S)-3-n-butylsulfonamidopiperidin)-1-cyclohexene-1-carboxylic acid. The compound of formula I has a great effect on inhibiting influenza virus, specially is highly active to virus strains which are resistant to Oseltamivir, and can be used as a broad-spectrum anti-influenza NA inhibitor effective to Tamiflu drug-resistance. Moreover, the compound is white and solid at room temperature, and can be crystallized and purified easily, which is beneficial for process scale-up in industry and suitable for producing in a large scale.

The present invention also discloses an injectable formulation of anti-influenza virus, which comprises the compound of formula I or a pharmaceutically acceptable salt thereof as an active ingredient.

It may be understood that the above injectable formulation may be injection liquid, a sterile powder for injection and a concentrated solution for injection, and may be used for intramuscular injection, intravenous injection and intravenous drip, which may be selected according to specific clinical demands, wherein the concentrated solution for injection may be diluted for intravenous drip before use. In the final solution for injection, the concentration of the active ingredient is 1 mg / mL - 300 mg / mL calculated in terms of the compound of formula I. The injectable formulation may be made into injection liquid in a packaging form of ampoule, glass vial, tube-type bottle, cartridge, penicillin bottle, prefillable syringe, plastic bottle, infusion bottle, plastic bag, or prefilled syringe or the like, and there is 0.5 - 500 ml of injection in each syringe or bottle, preferably 2 ml, 5 ml, 10ml, 20 ml, 50 ml, 100 ml, or 250 ml.

In one of the embodiments, the pharmaceutically acceptable salt of compound of formula I is lactate, hydrochloride, phosphate, acetate, malate, citrate, or aspartate. Water solubility of the compound of formula I is pH dependent, and thus its aqueous solution is not suitable for the clinical application due to its unstability. Therefore, the compound of formula I preferably reacts with lactic acid, hydrochloric acid, phosphoric acid, acetic acid, malic acid, citric acid, or aspartic acid to obtain salts, in order to achieve better stability and solubility and meet the demands of the clinical application.

In one of the embodiments, the pharmaceutically acceptable salt is lactate, wherein mole ratio of the compound of formula I to lactic acid is from 1:1.5 to 1:2.5. It has been found by the inventor that the optimal stability, solubility and body tolerance can be achieved in case that the compound of formula I is used in a form of its lactate in the mole ratio between above range through a large amount of experiments.

In one of the embodiments, the injectable formulation is sterile powder for injection which also comprises a pharmaceutically acceptable excipient. Preparing the injectable formulation into the sterile powder for injection can further improve the safety, effectiveness and stability of preparation. The sterile powder for injection may be prepared by solvent crystallization, spray drying process or freezing drying, or cryodrying after excipient is added. It may be understood that the pharmaceutically acceptable excipient comprises at least one of osmotic pressure modifier, pH modifier, solubilizer, antioxygen, bacteriostat, or filling agent.

It may be understood that the above excipient may be selected from the common excipient in the art according to the demands. For example, the osmotic pressure modifier comprises but is not limited to citric acid/citrate, acetate, mannitol, lactose, sorbitol, glucose, sodium chloride, potassium chloride, maltose, etc. The pH modifier comprises but is not limited to lactic acid, hydrochloric acid, phosphoric acid, acetic acid, malic acid, citric acid, or aspartic acid, etc., one or more of above modifiers may be added to obtain a proper pH value, preferably a range ranking from pH3 to pH6, most preferably from pH3.5 to pH5.0. The solubilizer comprises but is not limited to one or a mixture selected from more than two of Tween - 40, Tween - 60, Tween - 80, lecithin, soybean phospholipid, cetanol, octadecanol, poloxamer 188, polyoxyethylene castor oil, and sodium deoxycholic acid. The antioxygen comprises but is not limited to one or a mixture selected from more than two of sodium sulfite, sodium bisulfite, sodium pyrobisulfite, sodium thiosulfate, thiourea, butylated hydroxytoluene, butylated hydroxyanisole, and edetate disodium. The bacteriostat comprises but is not limited to one or a mixture selected from more than two of phenol, cresol, chlorocresol, chlorobutanol, benzyl alcohol, and thimerosal. The filling agent comprises but is not limited to one or a mixture selected from more than two of sodium chloride, sorbitol, and mannitol. Filling gas after purification may be added to prepareation packages according to the demands, wherein the filling gas comprises but is not limited to one or two of carbon dioxide and nitrogen.

In one of the embodiments, the sterile powder for injection is freeze-dried powder for injection, and the pharmaceutically acceptable excipient comprises filling agent, wherein the filling agent is at least one of mannitol, sorbitol and sodium chloride, and the ratio by weight of the filling agent to the compound of formula I or pharmaceutically acceptable salt thereof is in a range from 0.19:1 to 1.9: 1. It has been found by the inventor that the freeze-dried powder for injection prepared in above formula has the optimal stability, resolubility and body tolerance through a great number of experiments. It has been also found by the inventor that the type and the amount of the filling agent have an impact on the physical and chemical properties of the freeze-dried powder finally obtained, specifically when selecting the filling agents. Better performance will be achieved if sodium chloride is selected as the filing agent, and the weight ratio of the sodium chloride to the compound of formula I preferably is from 0.19:1 to 0.57:1, more preferably is from 0.25:1 to 0.57:1.

The present invention also discloses a method for preparing the aforesaid injectable formulation of anti-influenza virus, comprising steps: an amount of the compound of formula I and an appropriate amount of water for injection were added, followed by an organic acid or inorganic acid such that the compound of formula I was reacted with the organic acid or inorganic acid completely and dissolved, a predetermined amount of a pharmaceutically acceptable excipient was subsequently added, mixed, then the water for injection is added until a predetermined volume was reached, and filtered to obtain the final product.

It may be understood that according to preparation requirement of the injectable formulation in the art, after the liquid medicine is prepared, sterile filtration or high-temperature sterilization is still required, an aseptic method is used to distribute the liquid medicine into aseptic and clean packages with prescribed loading volume, thermally or tightly seal them, and perform a leakage detection by a reduced pressure method or other appropriate methods.

The present invention also discloses a method for preparing the aforesaid injectable formulation of anti-influenza virus, comprising the following steps:
preparation of solution: an amount the compound of formula I and an appropriate amount of water for injection were added, followed by an organic acid or inorganic acid such that the compound of formula I was reacted with the organic acid or inorganic acid completely and dissolved, a predetermined amount of a pharmaceutically acceptable excipient was subsequently added, mixed, a predetermined amount of a pharmaceutically acceptable excipient was subsequently added, mixed, and filtered; and
freeze-drying: the injectable solution prepared above was distributed and placed in a freeze-drying device, and freeze-dried to obtain the final product.

The above freeze-dried powder for injection is prepared by a freeze-drying method, being capable of ensuring the active ingredient is unchanged during the preparing process, and presents better stability and properties.

Likewise, according to the preparation requirement for freeze-dried powder for injection in the art, a leakage detection is performed by a reduced pressure method or other appropriate methods after the freeze-dried powder is prepared.

In one of the embodiments, the pharmaceutically acceptable excipient is sodium chloride in the step of solution preparing, wherein ratio by weight of the added excipient to the compound of formula I is from 0.19:1 to 0.57:1, preferably is 0.25:1 to 0.57:1;
in the step of freeze-drying process, the technical conditions is as follows: the temperature of shelves was set to -30 to -50 °C for 3 to 5 hours;
vacuuming was then conducted, the temperature was gradually raised by 7 to 15 °C within 0.5 to 1.5 hours, and maintained for 1.5 to 2.5 hours;
the temperature was then gradually raised by 7 to 15 °C within 0.5 to 1.5 hours, and maintained for 2 to 4 hours;
the temperature was then gradually raised by 7 to 15 °C within 1.5 to 2.5 hours, and maintained for 2 to 4 hours;
the temperature was then gradually raised up to 0 °C within 0.5 to 1.5 hours, and maintained for 1.5 to 2.5 hours;
the temperature was then gradually raised up to 15 to 25 °C within 0.5 to 1.5 hours, and maintained for 1.5 to 2.5 hours; and
the temperature was then gradually raised up to 35 to 45 °C within 0.5 to 1.5 hours, and maintained for 4 to 10 hours.

The inventor has thoroughly considered the characteristics of various materials and has done many experiments on conditions such as freezing rate, determination of object temperature, selection of vacuum degree, etc., to find that a plumpy, white, smooth, porous solid compound that is the freeze-dried powder with optimal properties can be obtained by above process condition.

The present invention further discloses use of the aforesaid cyclohexene derivate or a pharmaceutically acceptable salt thereof in preparation of a medicament for anti-influenza virus.

Compared to the prior art, the present invention has the following beneficial effects:
A new cyclohexene derivate or a pharmaceutically acceptable salt thereof of the present invention is a new broad-spectrum anti-influenza compound, which specially has higher activity against viral strains that are drug-resistant to Oseltamivir and Tamiflu.
An injectable formulation of anti-influenza virus of the present invention uses the aforesaid new cyclohexene derivate and has great inhibiting effects on influenza virus, specifically still has high activity on virus strains which are drug-resistant to Oseltamivir, and it may be used as a broad-spectrum anti-influenza NA inhibitor effective to Tamiflu drug-resistance, for treating influenza caused by influenza viruses, thereby it is a new broad-spectrum anti-influenza virus drug.
With regard to the poor solubility of the compound, a method for improving its solubility is provided to prepare an injectable formulation for effectively solving the clinical need of quick and timely medication of patients; a form of freeze-dried powder for injection is provided for convenient long-term storage and long-distance transport.
A method for preparing an injectable formulation of anti-influenza virus of the present invention is capable of preparing injection liquid or a freeze-dried powder for injection by appropriate methods after thoughtfully considering the active ingredients and the properties of an excipient, which not only ensures safety and effectiveness of the injections, but also improves the stability of the injections to meet the needs of clinical application.

### Description of Drawings

Figure 1 shows pictures of culture dishes in a plaque assay for measuring virus titer of Embodiment 2;
   wherein: A, B, C are pictures stained by crystal violet after being infected with PR8 virus for 3 days; D, E, F are pictures stained by immunoassay after being infected with H274Y virus for 5 days.
Figure 2 - A is a graph of weight loss on mice infected with influenza virus PR8 of Embodiment 2;

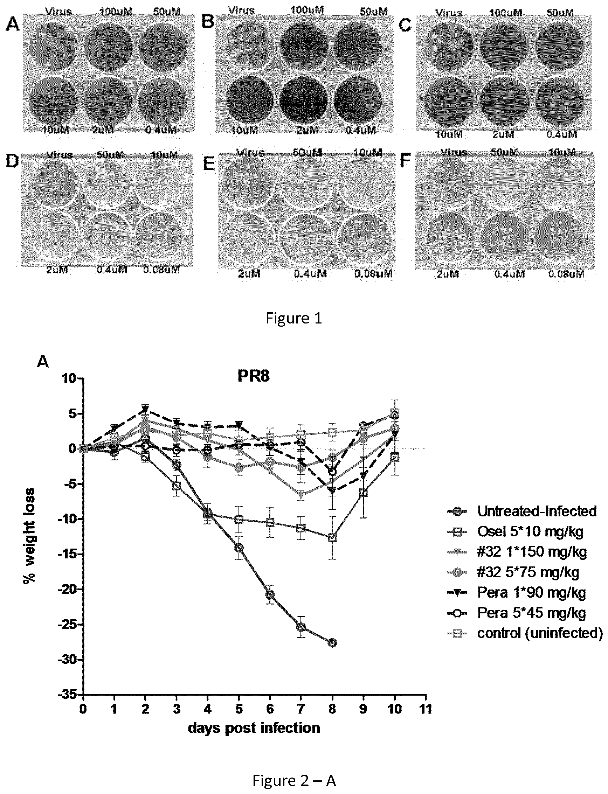

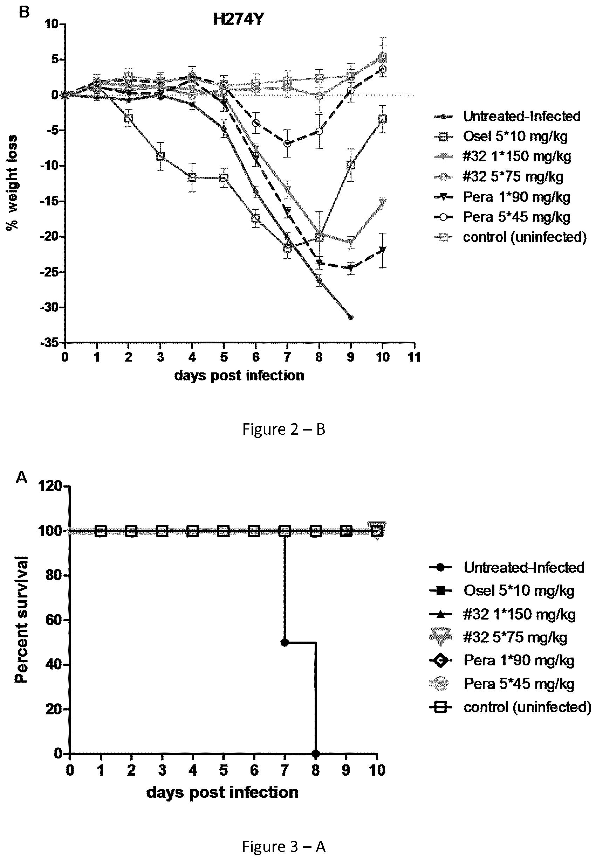

Figure 2 - B is a graph of weight loss on mice infected with influenza virus H274Y-PR8 of Embodiment 2;

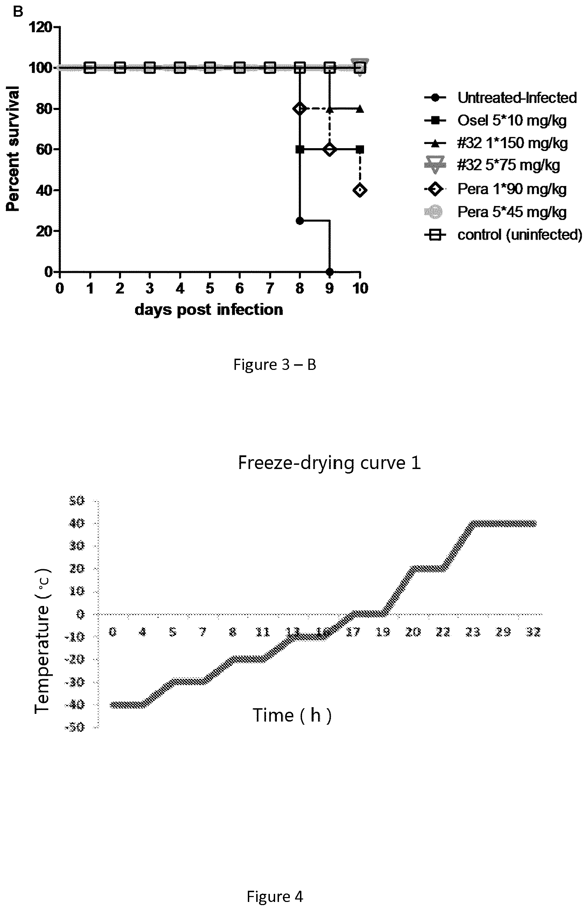

Figure 3 - A is a graph of survival rate of mice infected with influenza virus PR8 of Embodiment 2;
Figure 3 - B is a graph of survival rate of mice infected with influenza virus H274Y-PR8 of Embodiment 2;
Figure 4 is a freeze-drying curve 1 of Embodiment 4;

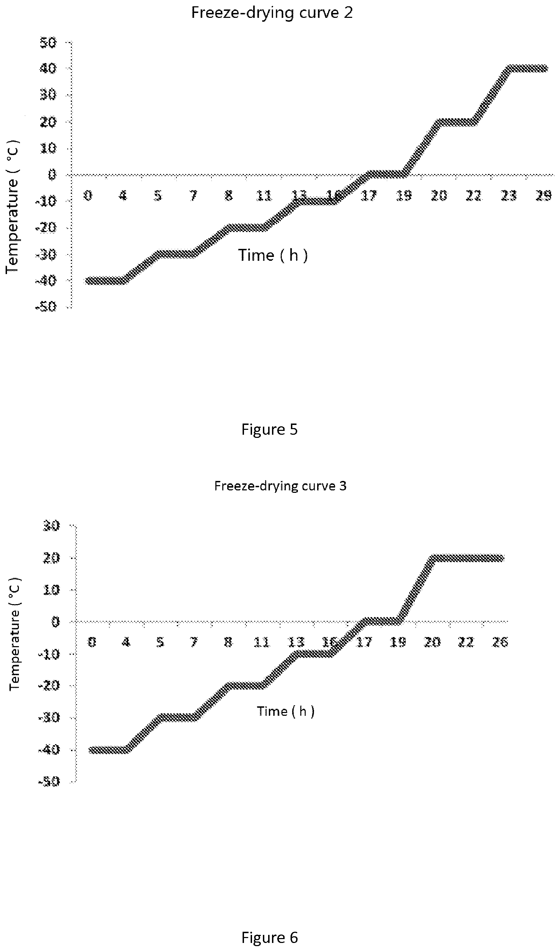

Figure 5 is a freeze-drying curve 2 of Embodiment 4;
Figure 6 is a freeze-drying curve 3 of Embodiment 4.

### Detailed Description of the Invention

The present invention will be described in more detail hereinafter with reference to accompanied figures in order to be better understood. However, the present invention may be achieved in a variety of forms and is not limited to the embodiments described herein. In contrast, the purpose of providing these embodiments is to enable a more thorough understanding for disclosed details of the present invention.

All of the techniques and terms used in the present invention have the same meanings as those understood by one skilled in the art unless otherwise defined. The terms used in specification of the present invention is only in a purpose of describing the specific embodiments, without aiming to make restriction on the present invention.

### Embodiment 1

Preparation of (3R,4R,5S)-4-acetamido-5-guanidino -3-((3R)-3-n-butylsulfonamidopiperidin)-1-cyclohexene-1-acrboxylic acid.

### a) Preparation of ethyl (3R,4S,5S)-4-acetamido-5-azido-3-((3R)-3-n-butylsulfonamidopiperidin)-1-cyclohexene-1-carboxylate.

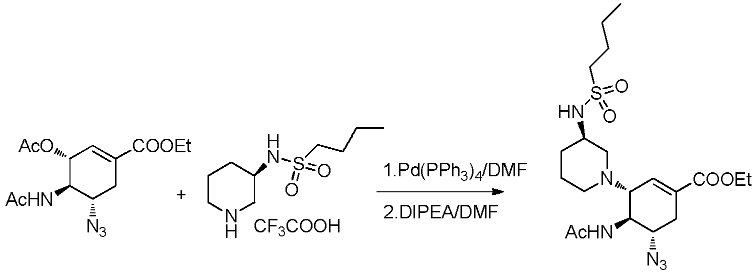

The preparation of ethyl (3R,4R,5S)-4-acetamido-5-azido-3-acetoxyl-1-cyclohexene -1-carboxylate refers to a method disclosed by J.Am. Chem. Soc., 1997(119):681-690.

An amount of 20 mmol of ethyl (3R,4R,5S)-4-acetamido-5-azido-3-acetoxyl-1-cyclohexene-1-carboxylate and 1 mmol of tetrakis (triphenylphosphine) palladium were added to a dry twin-neck flask, the air in the system was replaced with nitrogen gas twice, an amount of 40 mL of redistilled DMF was added with an injector and stirred well, an amount of 40 mmol of DIPEA (N,N-Diisopropylethylamine) was then added, and stirred until cooled to 0 °C, DMF solution (40 mL) of (R)-3-(n-butylsulfonamido)piperidin trifluoroacetate was gradually dripped and stirred at 0 °C for 20 minutes after the dripping, placed in an oil bath to react for 1 hour, gradually cooled it to 0 °C once the TLC (DCM : MeOH = 10 :1) indicated the reaction was completed, water was slowly dripped in the identical volume, stirred well and extracted with EA, dried directly through evaporation, and DMF is extracted by using an oil pump to obtain a brown viscous object. After column purification (MeOH : DCM = 1 : 50) and elution, a yellow-white powder solid of 4.37 g was obtained , that is the product of ethyl (3R,4R,5S)-4-acetamido-5-azido-3-((3R)-3-n-butylsulfonamidopiperidin) -1-cyclohexene-1-carboxylate, Y = 46.4%.

The characterization analysis of the above product was:
¹H-NMR(400MHz,CDCl₃):δppm6.810(s,1H,NH),5.529-5.506(d,1H,2-CH),4.233-4.1 99(q,2H,COOCH₂CH₃),4.067-3.991(m,1H,4-CH),3.655-3.589(m,2H,CH₂CH₂CH₂CH₃),3.3 58-3.334(m,1H,3-CH),3.019-3.001(m,1H,NCH₂CH),2.985-2.979(d,2H,NCH₂CH,NCH₂CH ₂CH₂),2.947-2.904(m,2H,NCH₂CH ,NCH₂CH₂CH₂),2.775-2.749(m,1H,5-CH),2.654-2.630(m,1H,6-CH),2.337-2.267(m,2H,6-CH,NCH₂CH₂CH₂),2.111(s,3H,COCH₃),1.819-1.744(m,2H,CH₂CH₂CH₂CH₃),1.721-1.701( m,1H,NCH₂CH₂CH₂),1.689-1.664(m,1H,NCH₂CH₂CH₂),1.621-1.598(m,1H, NCH₂CH₂CH₂),1.484-1.427(m,2H,CH₂CH₂CH₂CH₃) ,1.328-1.259(t,3H,COOCH₂CH₃),0.970-0.934 (t,3H,CH₂CH₂CH₂CH₃).
ESI-MS m/z: 471.6 (M+H)⁺

### b) Preparation of ethyl (3R,4R,5S)-4-acetamido-5-amino-3-((3R)-3-n-butylsulfonamidopiperidin)-1-cyclohexene-1-carboxylate.

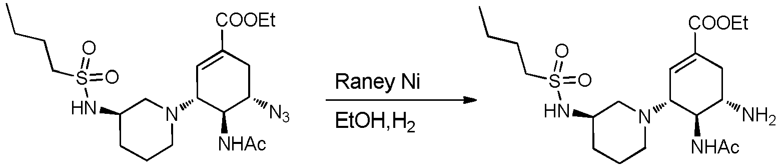

An amount of ethyl(3R,4R,5S)-4-acetamido-5-azido-3-((3R)-3-butylsulfonamidopiperidin) -1-cyclohexene-1-carboxylate was added to a single-neck flask, Raney Ni in catalytic amount and 40 mL of absolute ethyl alcohol were then added and stirred well, the air in the system was replaced with hydrogen gas, followed by continuing stirring at room temperature for 2 hours, and after the TLC (DCM : MeOH = 10 :1) indicated the reaction was completed , Raney Ni with diatomite was filtered out, the solvent was then evaporated, purifying (DCM : MeOH = 5 : 1) by column, to obtain a white foam solid, that is the product of ethyl (3R,4R,5S)-4-acetamido-5-amino-3-((3R)-3-n-butylsulfonamidopiperidin)-1-cyclohexene-1-carboxylate.

The characterization analysis of the above product was:
¹H-NMR(400MHz,CDCl₃):δppm6.773(s,1H,NH),6.248-6.225(d,1H,2-CH),5.198-5.1 80(m,1H,4-CH),4.207-4.156(q,2H,COOCH₂CH₃),3.894-3.821(m,1H,3-CH),3.708-3.656(t ,2H,CH₂CH₂CH₂CH₃),3.539-3.532(m,1H,5-CH),3.181-3.159(m,1H,NCH₂CH),3.001-2.920 (d,1H,NCH₂CH),2.834-2.799(m,2H,NCH₂CH₂CH₂),2.738-2.709(m,1H,NCH₂CH),2.608-2. 588(m,1H,6-CH),2.281-2.236(m,1H,6-CH),2.078(s,3H,COCH₃),2.028-1.998(m,1H,NCH₂ CH₂CH₂),1.798-1.724(m,2H,CH₂CH₂CH₂CH₃),1.712-1.701(m,1H,NCH₂CH₂CH₂),1.692-1. 598(m,2H,NCH₂CH₂CH₂),1.496-1.484 (m,2H,CH₂CH₂CH₂CH₃),1.296-1.212(t,3H,COOCH₂CH₃),0.998-0.956 (t,3H,CH₂CH₂CH₂CH₃).
ESI-MS m/z: 445.6 (M+H)⁺

### c) Preparation of ethyl (3R,4R,5S)-4-acetamido-5-(2,3-bis(tert-butyloxycarbonyl) guanidino)-3-((3R)-3-n-butylsulfonamidopiperidin)-1-cyclohexene-1-carboxylate.

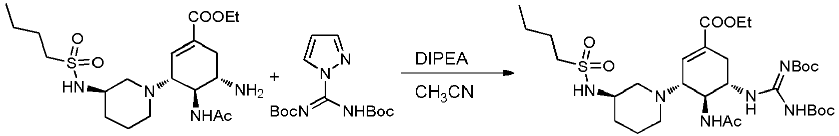

An amount of 0.5 mmol of ethyl (3R,4R,5S)-4-acetamido-5-amino-3-((3R)-3-n-butylsulfonamidopiperidin)-1-cyclohexene-1-carboxylate was added to a single-neck flask, 10 mL of acetonitrile was then added and stirred well at room temperature, 1. 5 mmol of DIPEA and 0.5 mmol of N, N'-bis-BOC-1*H*-1-guanidino pyrazole were then added, followed by stirring at room temperature for 3 hours, after the TLC (PE : EA = 1 :1) indicated the reaction was completed , 20 ml of water was added, extracted with EA, evaporated after drying with anhydrous sodium sulfate, and performed purification by column (PE : EA = 3 :1) to obtain white solid, that is the product of ethyl (3R,4R,5S)-4-acetamido-5-(2,3-bis(*tert*-butyloxycarbonyl) guanidino)-3-((3R)-3-n-butylsulfonamidopiperidin)-1-cyclohexene-1-carboxylate. ESI-MS m/z: 687.9 (M+H)⁺

### d) Preparation of (3R,4R,5S)-4-acetamido-5-(2,3-bis(tert-butyloxycarbonyl) guanidino)-3-((3R)-3-n-butylsulfonamidopiperidin)-1-cyclohexene-1-carboxylic acid.

An amount of 0.368 mmol of ethyl(3R,4R,5S)-4-acetamido-5-(2,3-bis(*tert*-butyloxycarbonyl) guanidino)-3-((3R)-3-n-butylsulfonamidopiperidin)-1-cyclohexene-1-carboxylate was added to a single-neck flask, 5.5 ml of 1,4-dioxane, 0.55 ml of water and 0.55 ml of 1N KOH aqueous solution were then added and stirred overnight at room temperature. After the TLC (PE : EA = 1 : 1) indicated that the reaction was completed, evaporated the solvent and dried it by using an oil pump, 5 ml of methanol is added to dissolve, pH was regulated to 5 with acid resin, followed by filtering, evaporating, and purifying by column (DCM : MeOH = 10 : 1), and eluting to obtain a white solid , that is the product of (3R,4R,5S)-4-acetamido-5-(2,3-bis(*tert*-butyloxycarbonyl) guanidino)-3-((3R)-3-n-butylsulfonamidopiperidin)-1-cyclohexene-1-carboxylic acid.

The characterization analysis of the above product was:
¹H-NMR(400MHz,MeOD):δppm6.833(d,1H,2-CH),4.263-4.237(m,1H,4-CH),4.191 -4.163(m,1H,3-CH),3.493-3.476(m,3H,5-CH ,CH₂CH₂CH₂CH₃),2.925-2.884(m,1H,NCH₂CH),2.582-2.528(d,2H,NCH₂CH,NCH₂CH₂CH₂) ,2.388-2.342(m,1H,NCH₂CH₂CH₂),2.245-2.230(m,1H,NCH₂CH),2.201-1.190(m,1H,6-CH ),2.154-2.143(m,1H,6-CH),1.929(s,3H,COCH₃),1.848-1.828(m,1H,NCH₂CH₂CH₂),1.772-1.717(m,3H,CH₂CH₂CH₂CH₃,NCH₂CH₂CH₂),1.621-1.598(m,2H,NCH₂CH₂CH₂),1.518(s,9H ,C(CH₃)₃),1.458(s,9H,C(CH₃)₃),1.347-1.281(m,2H,CH₂CH₂CH₂CH₃),0.976-0.939(t,3H,CH ₂CH₂CH₂CH₃).
ESI-MS m/z: 659.8 (M+H)⁺

### e) Preparation of (3R,4R,5S)-4-acetamido-5-guanidino-3-((3R)-3-n-butylsulfonamidopiperidin)-1-cyclohexene-1-carboxylic acid.

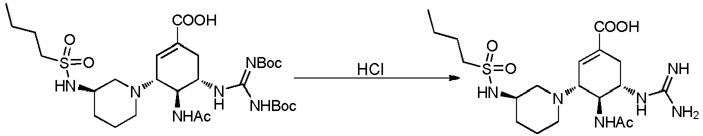

An amount of (3R,4R,5S)-4-acetamido-5-(2,3-bis(*tert*-butyloxycarbonyl) guanidino)-3-((3R)-3-n-butylsulfonamidopiperidin)-1-cyclohexene-1-carboxylic acid was added to a single-neck flask, dilute hydrochloric acid solution was dripped while keeping stirring at room temperature, followed by continuing stirring at room temperature until the reaction was finished, sodium hydroxide aqueous solution was slowly dripped until the solid was crystallized, performed filtration, and the filter residues was dried to obtain a white solid, that is (3R,4R,5S)-4-acetamido-5-guanidino-3-((3R)-3-n-butylsulfonamidopiperidin)-1-cyclohexene-1-carboxylic acid.

The purification of the compound of formula l: a rough product obtained in the embodiment was mixed with water and isopropanol, heated up to 50 °C, stirred evenly, then naturally cooled to crystallize, and filtered to obtain a refined product.

The characterization analysis of the above product was:
¹H-NMR(400MHz,D₂O):δppm6.814(d,1H,2-CH),4.473-4.447(m,1H,4-CH),4.435-4. 424(m,1H,3-CH),3.989-3.922(m,1H,5-CH ),3.670-3.644(m,2H,CH₂CH₂CH₂CH₃),3.594-3.541(m,1H,NCH₂CH),3.289-3.251(m,2H,N CH₂CH,NCH₂CH₂CH₂),2.388-2.3423.030-2.975(m,1H,NCH₂CH₂CH₂),2.511-2.439(m,1H, NCH₂CH),2.254-2.226(m,1H,6-CH),2.165-2.143(m,1H,6-CH),2.095(s,3H,COCH₃),1.998-1.898(m,1H,NCH₂CH₂CH₂),1.745-1.706(m,3H,CH₂CH₂CH₂CH₃,NCH₂CH₂CH₂),1.484-1.42 9(m,2H,NCH₂CH₂CH₂), 1.200-1.165(m,2H,CH₂CH₂CH₂CH₃),0.945-0.908(t,3H,CH₂CH₂CH₂CH₃).
ESI-MS m/z: 459.6 (M+H)⁺.

### Embodiment 2

### A. Activity assay for influenza virus neuraminidase

Experimental materials: NA (neuraminidase) solution: allantoic fluid of chicken embryo infected by influenza virus
Enzyme catalyzed reaction system:
330 mmol/L of MES buffer solution (pH3.5)
200 µmol/L of fluorogenic substrate MUNANA
4 mmol/L of CaCl₂ solution
Stop buffer: 14 mmol/L of NaOH solution (14 mmol/L, 83% ethanol)
Oseltamivir: 1 mmol/L
Peramivir: 1 mmol/L
Compared compound: compound of the following formula II, 1 mmol/L

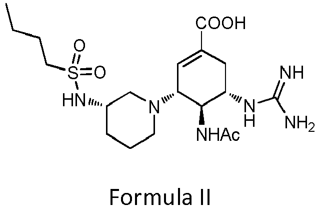

Enzyme activity assay:

| | |
|---|---|
| NA solution (with different diluted concentration) | 40 µl |
| MES (330 mmol/L) : | 10 µl |
| CaCl₂ (4 mmol/L) : | 10 µl |
| MUNANA (200 µlmol/L) : | 10 µl |
| H₂O: | 3 µl |

They were mixed together and incubated for 15 minutes, and 150 µl of the stop buffer was added:
EX=355 nm XM=460 nm

Note: it is possible to detect a dynamic curve of fluorescence values without addition of the stop buffer, in order to verify whether the enzyme reaction system works normally.

Experiment operation for screening a NA inhibitor is as follows:
1. Oseltamivir, Peramivir or testing compounds compound to be tested (the compound of formula I, II) was diluted in proportion of 1:10, 1:100, 1:1000, 1:10000.
2. reaction processes: 30 µl of NA solution was mixed with 10 µl of Oseltamivir, Peramivir or compound to be tested and incubated for 30 minutes, following ingredients were added: 10µl of MES (330 mmol/L), 10 µl of CaCl₂(4 mmol/L), 10 µl of MUNANA (200 µlmol/L), and 3 µl of H₂O. After they were mixed well and incubated for 15 minutes, 150 µl of the stop buffer was added, mixed well, finally, the fluorescence values were detected (EX=355nm XM=460nm).
3. data analysis: analyzing GraphPad Prism Demo.

The assay results of the activity of targeted compounds are shown in the table below:

**Table1. Activity of compounds**

| COMPOUND STRUCTURE | WIDE-TYPE IC₅₀ (nM ) | DRUG-RESISTANT MUTANT IC₅₀ (nM) |
|---|---|---|
| Compound of formula I | 0.5002 | 1.695 |
| Compared compound | 0.8671 | 8.031 |
| Oseltamivir phosphate | 0.7654 | 269.4 |
| Peramivir | 0.09845 | 26.93 |

It can be seen from the above results that the compound of formula I shows great inhibiting effect on H274Y drug-resistant mutant NA and its inhibiting effect is better than the compared compound (isomer of the compound of formula I, derived from Patent No. CN201310130035.2), far more better than Oseltamivir phosphate and Peramivir.

### B. Plaque assay for measuring virus titer

### 1. Experimental materials:

A/Puerto Rico/8/34 (H1N1) (PR8), H274Y virus, Madin-Darby canine kidney (MDCK) cells, DMEM (Gibco, Cat#C11965500BT), penicillin- streptomycin double antibody (Gibco, Cat#C11875), Trypsin (including 0.25%EDTA) (Gibco, Cat#25200-056), BSA (Merck, Cat#126575), Agrose (Biowest, Cat#111860), Triton X-100 (Amresco, Cat#9002931), PR8 mouse hyper-immune serum, goat-anti mouse-lgG-HRP secondary antibody (BOSTER, Cat#BA1050), AEC Staining kit (Sigma, AEC101), crystal violet (MBCHEM, Cat#548629), DPBS (C14190500BT, life technology), MOD EAGLE MED (MEM) (11935046, life technology), TWEEN -20 (P1379-6, SIGMA)
compound I to be tested: 1 mmol/L; Oseltamivir: 1 mmol/L; Peramivir : 1 mmol/L.

### 2. Experimental Method

A culture medium where a great quantity of MDCK cells had grown was sucked and removed, washed once with PBS, and then 1 ml of Trypsin was added to digest for 7 minutes at 37 °C, 5 ml of the culture medium was further added to stop digestion, slightly blew and flapped to make the cells scattered. The amount of the cells was counted by blood counting chamber and the cells were diluted, to obtain 1 X 10⁶ cells per ml, followed by transferring 1 ml to each hole of a six-hole culture plate, and cultured at 37 °C with 5% CO² for 24 hours.

DMEM virus-diluents including 0.3% BSA was prepared. Small tubes with distributed PB8 virus or H274Y virus, which were stored in a - 80 °C freezer, were taken out and unfrozen on the ice. The prepared virus diluents were diluted to obtain required titer and volume of viruses so that there were nearly 50 plaques in each virus control hole.

The culture medium was sucked out from the six-hole cellular culture plate, washed twice with PBS, and PBS was then removed. The virus infection liquid was added, and the cells were cultured in an incubator at 37 °C for 2 hours.

An amount of 1.6 % agarose gel was dissolved in a microwave, and placed into a water bath at 40 °C for heat preservation. After the viruses had finished their attachment, the virus infection liquid was sucked and removed, washed once with PBS, the prepared agarose culture medium (with ingredients of 1 x MEM, 0.3% BSA, 1 x PS antibiotic, 1 µg/mL TPCK pancreatin, compound to be tested with final concentration, 0.8% agarose gel) was added, 1 mL/hole, followed by culturing in an incubator at 37 °C. After 3 - 5 days, a morphology and an amount of plaques were observed.

The cell plate was placed on the ice for cooling after obvious plaques appeared in the virus control hole, and the top agarose gel layer was removed after 15 minutes. Different stain methods were chosen according to the properties of the plaques of virus. PR8 virus may form larger plaques so that crystal violet staining was selected; H274Y virus may form smaller plaques so that an immunostaining method was selected.

Crystal violet staining was performed as below: after removing the gel, 1 ml/hole of crystal violet staining solution (0.5% crystal violet in formalin solution) was added, left at room temperature for 15 minutes, and the staining solution was then removed, washed cleanly with water, and the plaques were then observed and counted.

3.5.2 immunosatining method was performed as below: after removing the gel, 500 µl of 4% paraformaldehyde was added for each hole to fix the cells for 15 minutes, 0.1% Trition - X100 solution was added to disrupt the membrane for 15 minutes; the Trition - X 100 solution was sucked out, and the cell plate was washed twice by using 500 µL/hole of PBST solution (1 x PBS added into 0.05% Tween - 20); 500 µL of mouse-anti PR8 - hyper - immune serum diluted in 1 :1000 was then added, followed by gently shaking the culture plate at room temperature for 1 - 2 hours, primary antibodies were recycled, the cell plate was washed three times with PBST, 3 minutes each time; an amount of 500 µL/hole of Goat-anti mouse - secondary antibodies diluted in 1:2500 was added, followed by gently shaking at room temperature for 1 to 2 hours, secondary antibodies were recycled, the cell plate was washed five times with PBST, 3 minutes each time; AEC staining buffer solution was added with 300 µL/hole to rest in a dark place at room temperature for 20 minutes; and the plaques were observed and taken photographs as shown in figure 1, and counted.

From the figure 1, the compound to be tested and the compared compound effectively inhibit the plaque formation of PR8 and H27R6. Specifically, A, B and C are pictures stained by crystal violet after being infected with PR8 virus for 3 days; D, E and F are pictures stained by immunization method after being infected with H274Y virus for 5 days; A, D shows the inhibition of the compound of formula I against virus plaque; B, E shows inhibition of the control compound of Peramivir against virus plaques; C, F shows inhibition of the control compound of Oseltamivir against virus plaques. The concentration of the compounds are labeled above and below the cell hole, and " Virus " represents a virus control group without the compounds.

GraphPad Prism 5 software was used to carry on non-linear regression analysis of the obtained data, to obtain IC₅₀ values, shown in the below table.

**Table 2. Inhibiting effect in vitro of a compound to be tested, Peramivir and Oseltamivir against influenza virus replication**

| VIRUS | IC₅₀ (µM) | | |
|---|---|---|---|
| | COMPOUND TO BE TESTED OF FORMULA I | Peramivir | Oseltamivir |
| PR8 | 0.2525 | 0.0176 | 0.1329 |
| H274Y | 0.1306 | 0.6432 | 15.03 |

The results indicate that the half maximal inhibitory concentration (IC₅₀) of the compound to be tested against plaque formation of H274Y is 0.13 µM), about 115 times lower than the IC₅₀ of Oseltamivir against plaque formation of H274Y; about 5 times lower than the IC₅₀ of Peramivir against plaque formation of H274Y. The half maximal inhibitory concentration (IC₅₀) of the compound to be tested against plaque formation of PR8 is 0.25 µM), similar to the IC₅₀ (0.13 µM) of Oseltamivir. In conclusion, the compound to be tested has a significantly better inhibiting effect on NA of drug-resistant influenza virus strain than Oseltamivir, and likewise has a great inhibiting effect on sensitive influenza viruses.

### C. Antiviral activity, protective effect of the compound to be tested on mice infected with influenza virus PR9, H274Y.

### 1. Experimental materials:

A/Puerto Rico/8/34(H1N1)(PR8), H274Y virus, BALB/c mice, phosphatic buffer solution (PBS), isoflurane, compound to be tested (#32) (that is the compound of formula I), Oseltamivir Peramivir.

### 2. Experimental Method:

An amount of twice titer volume (2M) of LD₅₀ of PR8 and H274Y-PR8 influenza virus were used to infect mice respectively. The test was conducted in groups of compound to be tested (#32), groups of Peramivir (Pera), a group of Oseltamivir (Osel) as well as infected control group and normal control group. The mice were anesthetized to a mild extent by using isoflurane, and 40µl of influenza viruses solution of twice titer volume (2M) of LD₅₀ was dripped into the nasal cavity. For the treated groups administering compound to be tested (#32), the mice were administered through thigh-intramuscular injection (each side of thigh muscle, 50 µl/side) in a dosage of 150 mg/kg once after being infected for 24 hours; the mice were successively administrated five times in a dosage of 75 mg/kg after being infected for 24 hours, once daily. For the treated groups administering Pera drug, the mice were administered through thigh-intramuscular injection in a dosage of 90 mg/kg once after being infected for 24 hours; the mice were successively administrated five times in a dosage of 45 mg/kg after being infected for 24 hours, once daily. For the treated group administering Osel drug, the mice were successively administrated five times through intragastrically administering in a dosage of 10 mg/kg with intragastric administration needles (200 µl/each) after being infected for 24hours, once daily.

The morbidity and mortality of the mice were observed within 10 days after being infected, the mice were weighted to draw a graph of weight loss (Figure 2 -A) and a graph of survival rate (Figure 3 - A).

For the untreated mice group which was infected with PR8 virus, the mice presented disease symptoms such as depression, disheveled leather, curved back and shiver, etc., after being infected for 3 days,. It can be seen from the Figure 2 - A that the weight of the mice in this group continually decreased after being infected for 3 days, and all of the mice died in the 8^{th} day after being infected (Figure 3- A).

For the treated group administering Osel intragastrically, the mice also represented obvious disease symptoms. After being infected for 8 days, the mice had about 15% of weight loss in average. Subsequently, the mice started to recover, and their weight almost returned to the level before infection at the 10^{th} day since being infected.

Neither the mice in the treated group administering compound to be tested (#32) once in a dosage of 150 mg/kg and the treated group administering compound to be tested (#32) 5 times in a dosage of 75 mg/kg appeared obvious disease symptoms. The weight of the mice slightly decreased on the 7^{th} day since being infected, and subsequently was regained. None of the mice in the treated group died (Figure 3-A).

An amount of twice titer volume (2M) of LD₅₀ of NAI drug-resistant mutant stain H274Y - PR8 virus was used to infect mice. For different groups, the mice were administered the compound to be tested (#32) and Pera through intramuscular injection, and were administered intragastrically Osel respectively, after being infected for 24 hours.

The mice in the untreated group infected with H274Y-PR8 virus appeared disease symptoms such as depression and fatigue on the 4^{th} day after infection, and the body weight of the mice began to decrease significantly on the 5^{th} day after infection (Figure 2 - B). All the mice died in the 9^{th} day (Figure 3 - B).

The infected mice were administered 10 mg/kg of Osel per day, after being administrated for 5 days, the mice still showed obvious disease symptoms, and 2 mice died on the 8^{th} day after infection. The mortality rate was 40% (Figure 3 - B).

After the infected mice were administered compound to be tested (#32) in a dosage of 75 mg/kg for 5 days, the treated mice showed no symptoms of any diseases, and its body weight was basically the same as that of the uninfected control mice. After the mice were administered Pera in a dosage of 45 mg/kg for 5 days, they had mild disease symptoms and their body weight decreased by about 6% on the 7^{th} day after infection (Figure 2-B). After the mice were administered the compound to be tested (#32) once in a dose of 150 mg/kg, their body weight still decreased significantly after infection, one mouse died on the 8^{th} day after infection, and the mortality rate was 20%. After the mice were administered Pera once in a dosage of 90 mg/kg, they showed obvious disease symbols and significant weight decline (Figure 2 - B), one mouse died on each of the 8^{th}, 9^{th}, and 10^{th} day after infection, and the mortality rate was 60% (Figure 3 - B).

It can be seen from the above test results that the compound disclosed in the present invention shows good inhibitory activity against influenza virus, especially, it shows high inhibitory activity against the neuraminidase of an oseltamivir-resistant influenza virus strain, and the weight of the mice shows its application prospects in the preparation of anti-influenza drugs, thus it is expected to be developed as a new drug.

### Embodiment 3

### A. Solubility test of different salts of the compound of formula I.

The compound of formula I was reacted with different acids such as lactic acid, hydrochloric acid, phosphoric acid, acetic acid, malic acid, citric acid, or aspartic acid for salt formation, the solubilities of different salts of the compound of formula I have been investigated, and the results are shown in the following table.

**Table 3. Solubility of different salts of the compounds of formula I**

| COMPOUND TYPE | THE MOLAR RATIO OF THE COMPOUND OF FORMULA I TO ACID | SOLUBILITY | PH VALUE OF THE SOLUTION |
|---|---|---|---|
| THE COMPOUND OF FORMULA I | / | 2.5 | 7.8 |
| THE LACTATE OF THE COMPOUND OF FORMULA I | 1:2 | 194 | 3.8 |
| THE HYDROCHLORIDE OF THE COMPOUND OF FORMULA I | 1 : 1.5 | 108 | 3.8 |
| THE PHOSPHATE OF THE COMPOUND OF FORMULA I | 1 : 1 | 160 | 3.5 |
| THE ACETATE OF THE COMPOUND OF FORMULA I | 1 : 2 | 35 | 3.9 |
| THE MALATE OF THE COMPOUND OF FORMULA I | 1 : 1 | 130 | 3.8 |
| THE CITRATE OF THE COMPOUND OF FORMULA I | 1 : 1 | 95 | 3.7 |
| THE ASPARATE OF THE COMPOUND OF FORMULA I | 1 : 2 | 35 | 4.5 |

It can be seen from the above results that the solubility of the salts formed by compound of the formula I and an acid such as lactic acid, hydrochloric acid, phosphoric acid, acetic acid, malic acid, citric acid or aspartic acid is greatly improved most of the solubilities are more than 100 mg/mL if the pH = 3.8, wherein when the molar ratio of the compound of formula I to lactic acid is about 1:2, the solubility of the salt is 194 mg/mL, the pH of the solution is 3.8; when the molar ratio of the compound of formula I to malic acid is about 1:1, the solubility is 130 mg/mL, the solution pH is 3.8; and when the molar ratio of the compound of formula I to acetic acid/aspartic acid substance is 1:2, the solubility is 35 mg/mL. Lactic acid is preferable.

### B. Solubility test of the lactate of the compound of formula I.

Based on the above studies, the solubilities of the salts formed by different amounts of lactic acid and the compound of formula I have been further researched. The results are shown in the following table.

**Table 4. Solubility of the lactate salt of the compound of formula I**

| THE MOLAR RATIO OF THE COMPOUND OF FORMULA I AND LACTIC ACID | pH VALUE | SOLUBILITY |
|---|---|---|
| 1 : 2 | 3.8 | 194 mg/mL |
| 1 : 1 | 4.0 | 92 mg/mL |
| 1 : 0.8 | 4.5 | 46 mg/mL |
| 1: 0.75 | 5.0 | 20 mg/mL |
| 1 : 0.5 | 6.0 | 8.1mg/mL |
| Non-lactic acid | 7.8 | 2.5 mg/mL |

The above results show that adding different proportions of the lactic acid does not change the pH of the solution too much, but has a big effect on the solubility, wherein the addition amount of lactic acid is proportional to the solubility, and the more the addition amount, , the higher the solubility. However, too much lactic acid may cause much lower pH, resulting in injection pain, which is not suitable for clinical application, thereby the optimal mole ratio of the compound of formula I and lactic acid is about 1:2.

### Embodiment 4

### 1. Selection of Prescription of freeze-dried powder for injection.

Different prescriptions were designed in the experiments, wherein different kinds of excipients such as sodium chloride, glucose, lactose, sucrose, sorbitol, mannitol and dextran, etc., were selected. The details are shown in the following table, the finished products after lyophilization have been observed to research the composition of the prescription.

| MATERIAL AND EXCIPIENTS | PRESCRIPTION 1 | PRESCRIPTION 2 | PRESCRIPTION 3 µl | PRESCRIPTION 4 | PRESCRIPTION 5 | PRESCRIPTION 6 | PRESCRIPTION 7 |
|---|---|---|---|---|---|---|---|
| Cyclohexene derivate (mg) | 265 | 265 | 265 | 265 | 265 | 265 | 265 |
| Lactic acid (mg) | 111 | 111 | 111 | 111 | 111 | 111 | 111 |
| Mannitol (mg) | 125 | NA | NA | NA | NA | NA | NA |
| Sorbitol (mg) | NA | 125 | NA | NA | NA | NA | NA |
| Dextran (mg) | NA | NA | 125 | NA | NA | NA | NA |
| Sucrose (mg) | NA | NA | NA | 125 | NA | NA | NA |
| Glucose (mg) | NA | NA | NA | NA | 125 | NA | NA |
| Sodium chloride (mg) | NA | NA | NA | NA | NA | 125 | NA |
| Lactose (mg) | NA | NA | NA | NA | NA | NA | 125 |
| Water for injection (ml) | 2.5 | 2.5 | 2.5 | 2.5 | 2.5 | 2.5 | 2.5 |

| | | | | | | | |
|---|---|---|---|---|---|---|---|
| Note: NA means no addition. | | | | | | | |

The above formulas were prepared according to the following method:

### A. Preparation of solution.

An amount of 3 ml of water for injection and the compound of formula I were added to a penicillin bottle, and stirred. Lactic acid was added to dissolve the drug completely, different fillers were then added, stirred till they were dissolved well. The solution was filtered and distributed for use.

### B. Freeze-drying.

### 1. Pre-freezing.

The temperature of shelves was set to -40 °C, and drug temperature was maintained below -40 °C for several hours.

### 2. Freeze-drying.

Vacuuming was carried out, and drying operation was then conducted according to the following freeze-drying process conditions.

As shown in Figure 4, the freeze-drying conditions are as follows: the temperature of shelves was set to -40 °C and maintained below -40 °C for 4 hours, and vacuuming was then performed. The temperature was gradually raised up to -30 °C within 1 hour, and maintained for 2 hours. The temperature was then gradually raised up to -20 °C within 1 hour, and maintained for 3 hours. The temperature was then gradually raised up to -10 °C within 2 hours, and maintained for 3 hours. The temperature was then gradually raised up to 0 °C within 1 hour, and maintained for 2 hours. The temperature was then gradually raised up to 20 °C within 1 hour, and maintained for 2 hours. The temperature was then gradually raised up to 40 °C within 1 hour, and maintained for 9 hours.

The properties of the finished freeze-dried powder for injection prepared by the above method were observed and subjected to a redissolving experiment. The experiment was accelerated for 10 days at a high temperature of 40 °C, and the properties and the impurity contents of the finished product were observed. The results are shown in the following table.

**Table 6. Impact of different prescriptions on finished products.**

| TESTING ITEMS | | PRESCRIPTION 1 | PRESCRIPTION 2 | PRESCRIPTION 3 | PRESCRIPTION 4 | PRESCRIPTION 5 | PRESCRIPTION 6 | PRESCRIPTION 7 |
|---|---|---|---|---|---|---|---|---|
| Properties of finished products | | White powder | White powder | White powder | White powder | White powder | White powder | White powder |
| Accelerated Experiment | Properties | White powder | White powder | White powder | White powder | White powder | White powder | White powder |
| | Inpurity (%) | 0.12 | 0.25 | 0.97 | 0.92 | 0.79 | 0.11 | 0.24 |
| Redissolving experiment | Clarity | complies | complies | complies | complies | complies | complies | complies |
| | Redissolving Time (min) | <1 | <1 | >5 | >3 | >3 | <1 | >3 |

It can be seen from the above results that the powder injections prepared by using sodium chloride, sorbitol and mannitol as a filler have better stability and redissolving properties.

### B. Selection of process of freeze-dried powder for injection.

In the experiment, different freeze-drying processes as shown in Figures 4, 5 and 6 were designed to research the effects of three different freeze-drying processes on the finished product. Details are shown as follows:

**Table 7. Comparison among freeze-drying processes.**

| TESTING ITEMS | | FREEZE-DRYING CURVE 1 | FREEZE-DRYING CURVE 2 | FREEZE-DRYING CURVE 3 |
|---|---|---|---|---|
| Properties of finished products | | White powder | collapse | collapse |
| Redissolving Experiments | clarity | complies | complies | complies |
| | Redissolving time (min) | <1 | <1 | <1 |

As can be seen from the above results, the powder injection prepared by the freeze-drying process shown in Figure 4 has a good appearance and redissolving property.

### Embodiment 5

An injectable formulation of anti-influenza virus has a prescription of 60 g of the compound of the formula I, 25 g of citric acid and 1200 ml of water for injection.

A method for preparing the above injectable formulation is provided as follows:
An amount of 60 g of the compound of formula I and 500 ml of water for injection were added together, and stirred; pH was adjusted to 3.5 to dissolve the compound by adding citric acid, followed by diluting to 1200 ml with water for injection, and performed rough filtering; 0.22 µm of a sterile filter membrane was used to filter, the solution was then ultrafiltered; an inspection was made, 5 ml of the solution was distributed into each bottle, a plug was pressed in, and the bottle was covered with an aluminum-plastic composite cover, performed packaging and an inspection, to obtain a finished product.

### Embodiment 6

An injectable formulation of anti-influenza virus has a prescription of 25 g of the compound of the formula I, 5.7 g of lactic acid and 1000 ml of water for injection.

A method for preparing the above injection solution is provided as follows:
An amount of 25 g of the compound of formula I and 200 ml of water for injection were added together, and stirred; pH was adjusted to 4.5 to dissolve the compound by adding lactic acid, followed by diluting to 1000 ml with water for injection, and performed rough filtering; 0.22 µm of a sterile filter membrane was used to filter, the solution was then ultrafiltered; an inspection was made, 10 ml of the solution was distributed into each bottle, a plug was pressed in, and the bottle was covered with an aluminum-plastic composite cover, performed packaging and an inspection, to obtain a finished product.

### Embodiment 7

An injection against influenza virus has a prescription of 2 g of the compound of the formula I, 400 mg of hydrochloric acid and 800 ml of water for injection.

A method for preparing the above injection solution is provided as follows:
An amount of 2 g of the compound of formula I and 500 ml of water for injection were added together, and stirred; pH was adjusted to 5.0 to dissolve the compound by adding hydrochloric acid, followed by diluting to 800 ml with water for injection, and performed rough filtering; 0.22 µm of a sterile filter membrane was used to filter, the solution was then ultrafiltered; an inspection was made, 100 ml of the solution was distributed into each bottle, a plug was pressed in, and the bottle was covered with an aluminum-plastic composite cover, performed packaging and an inspection, to obtain a finished product.

### Embodiment 8

A freeze-dried powder for injection against influenza virus has a prescription of 5.0 g of the compound of the formula I, 2.1 g of lactic acid, 1.8 g of sodium chloride, 0.3 g of activated carbon, and 60 ml of water for injection.

A method for preparing the above freeze-dried powder for injection is provided as follows:

### A. Preparation of solution.

After the raw materials of compound of the formula I and the excipient were sterilized, 60 ml of water for injection was added to 5.0 g of the compound of the formula I. The pH value was adjusted to 3.8 to dissolve the drug completely by slowly adding lactic acid, 1.8 g of sodium chloride and 0.3 g of activated carbon were added, and stirred for 30 minutes. After filtering, the solution was distributed into 20 bottles under aseptic conditions for use.

### B. Freeze-drying.

### 1. Pre-freezing.

The temperature of shelves was set to -40 °C, and the temperature of the drug solution was maintained below -40 °C for 4 hours.

### 2, Freeze-drying.

Vacuuming was carried out, and drying operation was then conducted according to the following freeze-drying process conditions.

As shown in Figure 4, the freeze-drying conditions are as follows: the temperature of shelves was set to -40 °C, and maintained below -40 °C for 4 hours, and vacuuming was then performed. The temperature was then gradually raised up to -30 °C within 1 hour, and maintained for 2 hours. The temperature was then gradually raised up to -20 °C within 1 hour, and maintained for 3 hours. The temperature was then gradually raised up to -10 °C within 2 hours, and maintained for 3 hours. The temperature was then gradually raised up to 0 °C within 1 hour, and maintained for 2 hours. The temperature was then gradually raised up to 20 °C within 1 hour, and maintained for 2 hours. The temperature was then gradually raised to up 40 °C within 1 hour, and maintained for 6 hours.

### Embodiment 9

A freeze-dried powder for injection against influenza virus has a prescription of 2.0 g of the compound of the formula I, 0.88 g of lactic acid, 0.8 g of sodium chloride, 100 mg of activated carbon, and water for injection added till 20 ml.

A method for preparing the above freeze-dried powder for injection is provided as follows:

### A. Preparation of solution.

After the raw material of compound of the formula I and the excipient were sterilized, 15 ml of water for injection was added to 2.0 g of the compound of the formula I. The pH was adjusted to 3.8 to dissolve the drug completely by slowly adding lactic acid, 0.8 g of sodium chloride and 100 mg of activated carbon were added, and stirred for 30 minutes. After filtering, the solution was distributed into 8 bottles under aseptic conditions, for freeze-drying.

### B. Freeze-drying

### 1. Pre-freezing.

The temperature of shelves was set to -40 °C, and the drug temperature was maintained below -40 °C for 4 hours.

### 2. Freeze-drying.

Vacuuming was carried out, and drying operation was then conducted according to the freeze-drying process conditions of Embodiment 8, to obtain a finished product.

### Embodiment 10

A freeze-dried powder for injection against influenza virus has a prescription of 500 mg of the compound of the formula I, 200 mg of lactic acid, 300 mg of mannitol, and 3 ml of water for injection.

A method for preparing the above freeze-dried powder for injection is provided as follows:

### A. Preparation of solution.

An amount of 3 ml of water for injection and 500 mg of the compound of formula I were added to a penicillin bottle, and stirred. The pH value was adjusted to 4.0 to dissolve the drug completely by adding lactic acid, 300 mg of mannitol was added, and stirred well till dissolving. After filtering, the solution is distributed into 2 bottles for use.

### B. Freeze-drying.

### 1. Pre-freezing.

The temperature of shelves was set to -40 °C, and the drug temperature was maintained below -40 °C for hours.

### 2. Freeze-drying.

Vacuuming was carried out, and drying operation was then performed according to the following freeze-drying process conditions.

Subsequently vacuuming was carried out, and the product was obtained after drying according to the freeze-drying process conditions of Embodiment 8.

### Embodiment 11

A freeze-dried powder for injection against influenza virus has a prescription of 5.0 g of the compound of the formula I, 2.0 g of lactic acid, 5 g of dextran, and 50 ml of water for injection.

A method for preparing the above freeze-dried powder for injection is provided as follows:

### A. Preparation of solution.

The Preparation method is as follows: 50 ml of water for injection and 5.0 g of a cyclohexene derivative were added to a penicillin bottle, and stirred. The pH was adjusted to 4.0 to dissolve the drug completely by adding lactic acid, 5 g of dextran, was added and stirred well till dissolving. After filtering, the solution was distributed into 20 bottles for use.

### B. Freeze-drying.

### 1. Pre-freezing.

The temperature of shelves was set to -40 °C, and drug temperature was maintained below -40 °C for 4 hours.

### 2. Freeze-drying.

Vacuuming was carried out, and drying operation was conducted according to the freeze-drying process conditions of Embodiment 8.

### Embodiment 12

According to requirements of stability test of the pharmaceutical preparations prescribed by the pharmacopoeia, a long-term research about the compound to be tested (the sample prepared according to Embodiment 9) was carried out, and the results are shown in the following table.

**Table 8 Long-term Test Results (25 °C)**

| MONTHS | APPEARANCE | pH VALUE | CLARITY | RELATED SUBSTANCE% | CONTENTS% | MOISTURE % |
|---|---|---|---|---|---|---|
| 0 | White powder | 4 | complies | 0.25 | 99.9 | <1% |
| 3 | White powder | 4.02 | complies | 0.38 | 100.3 | <1% |
| 6 | White powder | 3.98 | complies | 0.37 | 100.2 | <1% |
| 9 | White powder | 4 | complies | 0.39 | 99.5 | <1% |
| 12 | White powder | 4.05 | complies | 0.42 | 99.6 | <1% |
| 18 | White powder | 4.05 | complies | 0.45 | 98.9 | <1% |
| 24 | White powder | 4.04 | complies | 0.46 | 98.9 | <1% |

It can be seen from the above results that a bacterial endotoxin test and a sterility test are carried out for the above long-term reserved samples after being reserved for 12 months and 24 months respectively, and the results show that both the bacterial endotoxin and sterility of the samples are in compliance with the requirements. It means that the appearance color, pH value, clarity, related substances, contents and moistures of the samples still meet the requirements after the product has been reserved for 24 months under the packaging in the market.

The technical features of the above-described embodiments may be combined in any combination. For the sake of brief description, not all possible combinations of the technical features in the above embodiments are described herein. However, as long as there is no contradiction between the combinations of these technical features, they should be considered fall within the scope of this specification.

The above-described embodiments merely illustrate several embodiments of the present invention, and their descriptions are detailedly and specifically, but should not be understood as the limit to the scope of the present invention. It should be noted for one skilled in the art that a number of variations and modifications may be made without departing from the spirit and scope of the present invention. Therefore, the scope of the present invention should be determined by the appended claims.

## Claims

1. A cyclohexene derivative of formula I or a pharmaceutically acceptable salt or prodrug thereof:

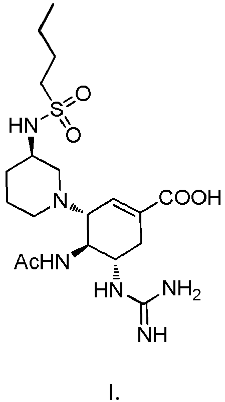

2. An injectable formulation of anti-influenza virus, wherein the injectable formulation comprises the compound of formula I or a pharmaceutically acceptable salt thereof as an active ingredient.

3. The injectable formulation of anti-influenza virus according to claim 1, wherein the pharmaceutically acceptable salt of the compound of formula I is lactate, hydrochloride, phosphate, acetate, malate, citrate, or aspartate.

4. The injectable formulation of anti-influenza virus according to claim 3, wherein the pharmaceutically acceptable salt is lactate and the mole ratio of the compound of formula I to lactic acid is from 1:1.5 to 1:2.5.

5. The injectable formulation of anti-influenza virus according to claim 2, wherein the injectable formulation is a sterile powder for injection and the sterile powder further comprises a pharmaceutically acceptable excipient.

6. The injectable formulation of anti-influenza virus according to claim 5, wherein the sterile powder for injection is a freeze-dried powder for injection, and the pharmaceutically acceptable excipient comprises a filler, the filler is at least one of mannitol, sorbitol, and sodium chloride, and the ratio by weight of the filler to the compound of formula I or a pharmaceutically acceptable salt thereof is from 0.19:1 to 1.9:1.

7. A method for preparing the injectable formulation of anti-influenza virus according to claim 2, comprising the following steps: an amount of the compound of formula I and an appropriate amount of water for injection were added, followed by an organic or inorganic acid such that the compound of formula I was reacted with the organic or inorganic acid completely and dissolved, a predetermined amount of a pharmaceutically acceptable excipient was subsequently added, mixed, then the water for injection was added until a predetermined volume was reached, and filtered to obtain the final product.

8. The method for preparing the injectable formulation of anti-influenza virus according to claim 6, comprising the following steps:
preparation of solution: an amount of the compound of formula I and an appropriate amount of water for injection were added, followed by an organic or inorganic acid such that the compound of formula I was reacted with the organic or inorganic acid completely and dissolved, a predetermined amount of a pharmaceutically acceptable excipient was subsequently added, mixed, a predetermined amount of a pharmaceutically acceptable excipient was subsequently added, mixed, and filtered; and
freeze-drying: the injectable solution prepared above was distributed and placed in a freeze-drying device, and freeze-dried to obtain the final product.

9. The method for preparing the injectable formulation of anti-influenza virus according to claim 8, wherein, in the step of solution preparation, the pharmaceutically acceptable excipient is sodium chloride added in a ratio by weight from 0.19:1 to 0.57:1 of the excipient and the compound of the formula I;
in the step of freeze-drying, the freeze-drying was carried out according to the following processes:
the temperature of shelves was set to -30 to -50 °C, and maintained for 3 to 5 hours;
vacuuming was then conducted, the temperature was gradually raised by 7 to 15 °C within 0.5 to 1.5 hours, and maintained for 1.5 to 2.5 hours;
the temperature was then gradually raised by 7 to 15 °C within 0.5 to 1.5 hours, and maintained for 2 to 4 hours;
the temperature was then gradually raised by 7 to 15 °C within 1.5 to 1.5 hours, and maintained for 2 to 4 hours;
the temperature was then gradually raised up to 0 °C within 0.5 to 1.5 hours, and maintained for 1.5 to 2.5 hours;
the temperature was then gradually raised up to 15 to 25 °C within 0.5 to 1.5 hours, and maintained for 1.5 to 2.5 hours; and
the temperature was then gradually raised up to 35 to 45 °C within 0.5 to 1.5 hours, and maintained for 4 to 10 hours.

10. Use of the cyclohexene derivative or a pharmaceutically acceptable salt thereof according to claim 1 in the preparation of a medicament for anti-influenza virus.
